# NEW EUROPEAN PATENT SPECIFICATION

(11) **EP 2 546 221 B2**
(45) Date of publication and mention of the opposition decision: **01.05.2024**
(45) Mention of the grant of the patent: 26.07.2017
(21) Application number: 12178198.3
(22) Date of filing: 03.01.2007
(51) Int. Cl.: C07C 17/25, C07C 21/18

(54) **Method for producing fluorinated organic compounds**
Verfahren zur Herstellung fluorinierter organischer Verbindungen
Procédé de production de composés organiques fluorés

(30) Priority: 03.01.2006 US 755485 P
(43) Date of publication of application: 16.01.2013
(62) Divisional of application: 07716229.5
(73) Proprietor: Honeywell International Inc., Morris Plains, NJ 07950 (US)
(72) Inventor: Merkel, Daniel, C., West Seneca, NY 14224 (US); Tung, Hsueh, Sung, Getzville, NY 14068 (US); Van der Puy, Michael, Amherst, NY 14221 (US); Ma, Jing, Ji, Morristown, NJ 07962-2245 (US); Dubey, Rajesh, Buffalo, NY 14210 (US); Light, Barbara, Niagra Falls, NY 14305 (US); Bortz, Cheryl, N Tonawanda, NY 14120 (US); Phillips, Steven, D., Buffalo, NY 14218 (US); Mukhopahyay, Sudip, Morristown, NJ 07962-2245 (US)
(74) Representative: Crooks, Elizabeth Caroline

(56) References cited:
- WO-A2-2005/012212
- WO-A2-2005/042451
- WO-A2-2007/079431
- US-A- 2 996 555
- HENNE, ALBERT L. ET AL: "Fluorinated derivatives of propane and propylene. VI", JOURNAL OF THE AMERICAN CHEMICAL SOCIETY, vol. 68, 1946, pages 496-497, XP002448570,
- PALETA, OLDRICH ET AL: "Synthesis of perfluoroallyl chloride and some chlorofluoropropenes", BULLETIN DE LA SOCIETE CHIMIQUE DE FRANCE, no. 6, 1986, pages 920-924, XP009088473,
- MARIA O. BURGIN ET AL: "UNIMOLECULAR REACTION KINETICS OF CF2CLCF2CH3 AND CF2CLCF2CD3: EXPERIMENTAL EVIDENCE FOR A NOVEL 1,2-FCL REARRANGEMENT PATHWAY", J. PHYS. CHEM. A, vol. 105, 2001, pages 1615-1621, XP002448571,
- HEARD et al.: "1,2-FCl Rearrangement as an Intermediate Step in the Unimolecular 1,3-HCl Elimination from Chlorofluoropropanes", J. Phys. Chem. A, vol. 105, 2001, pages 1622-1625,
- Safety data sheet for HCFC-245bb (Synquest Laboratories)
- Material safety data sheet for HCFC-244bb (Honeywell)

## Description

### BACKGROUND OF INVENTION

### (1) Field of Invention:

This invention relates to novel methods for preparing HFO-1234yf.

### (2) Description of Related Art:

Hydrofluorocarbons (HFC's), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze)) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkenes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions, Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts.

The preparation of HFO-1234yf from trifluoroacetylacetone and sulfur tetrafluoride has been described. See Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

Henne et al reported the synthesis of fluorinated derivatives of propane and propylene (J. Am. Chem. Soc., 68, 1946, 496-497). Paleta et al reported the synthesis of perfluoroallyl chloride and some chlorofluoropropenes (Bulletin de la Societie Chimique de France, 6, 1986, 920-924). Burgin et al reported the unimolecular reaction kinetics of CF2ClCF2CH3 and CF2ClCF2CD3 (J. Phys. Chem. A, 105, 2001, 1615-1621).

### SUMMARY

Applicants have discovered a method for producing HFO-1234yf, which comprises converting 1,1,1,2-tetrafluoro-2-chloropropane (HCFC-244bb) into 2,3,3,3-tetrafluoro-1-propene (HFO-1234yf);
wherein the reaction is carried out in a gas phase in the presence of a catalyst selected from the group consisting of carbon and/or metal based catalyst, nickel based catalyst, and palladium based catalyst, and wherein the reaction temperature is from 200°C to 800°C.

### DETAILED DESCRIPTION OF PREFERRED EMBODIMENTS

One beneficial aspect of the present invention is that it enables the production of HFO-1234yf using relatively high conversion and high selectivity reactions. Furthermore, the present methods in certain preferred embodiments permit the production of HFO-1234yf from relatively attractive starting materials. For example, 2-chloro-2,3,3,3-tetrafluoropropane is an advantageous starting material because it is relatively easy to handle, and can be produced by reference to the information provided in Sections I and II below.

### I. FLUORINATION OF THE COMPOUND OF FORMULA IA)

A compound of Formula (IA) (C(X)₂=CClC(X)₃ wherein each X is independently H or Cl) can be fluorinated to produce a compound of Formula (IAA) (C(X)₂=CClCF₃ wherein each X is independently H or Cl). In certain preferred embodiments, said compound(s) are fluorinated, preferably with HF in a gas phase, to produce HFO-1223xf. Preferably this gas phase reaction is at least partially catalyzed.

The preferred fluorination of the compound of Formula (IA) is preferably carried out under conditions effective to provide a Formula (IA) conversion of at least about 50%, more preferably at least about 75%, and even more preferably at least about 90%. In certain preferred embodiments the conversion is at least about 95%, and more preferably at least about 97%. Further in certain preferred embodiments, the conversion of the compound of Formula (IA) comprises reacting such compound under conditions effective to produce at least one compound of Formula (IAA), such as monochlorotrifluoropropene (preferably CF₃CCl=CH₂ (HFO-1233xf)) at a selectivity of at least about 50%, more preferably at least about 70%, more preferably at least about 80%, and even more preferably at least about 90%, with selectivities of about 95% or greater being achieved in certain embodiments.

In general, it is possible that the fluorination reaction step can be carried out in the liquid phase or in the gas phase, or in a combination of gas and liquid phases, and it is contemplated that the reaction can be carried out batch wise, continuous, or a combination of these.

For embodiments in which the reaction comprises a liquid phase reaction, the reaction can be catalytic or non-catalytic. Preferably, a catalytic process is used. Lewis acid catalyst, such as metal-halide catalysts, including antimony halides, tin halides, thallium halides, iron halides, and combinations of two or more of these, are preferred in certain embodiments. Metal chlorides and metal fluorides are particularly preferred. Examples of particularly preferred catalysts of this type include SbCl₅, SbCl₃, SbF₅, SnCl₄, TiCl₄, FeCl₃ and combinations of two or more of these.

In preferred gas phase fluorination Formula (IA) compounds, the reaction is at least partially a catalyzed reaction, and is preferably carried out on a continuous basis by introducing a stream containing the compound of Formula (IA) into one or more reaction vessels, such as a tubular reactor. In certain preferred embodiments, the stream containing the compound of Formula (IA) is preheated to a temperature of from 80°C to 400°C, more preferably from 150°C to 400°C, and in certain embodiments preferably 300°C, and introduced into a reaction vessel (preferably a tube reactor), which is maintained at the desired temperature, preferably from 80°C to 700°C, more preferably from 90°C to 600°C, even more preferably in certain embodiments from 400°C to 600°C, more preferably from 450°C to 600°C, where it is preferably contacted with catalyst and fluorinating agent, such as HF.

Preferably the vessel is comprised of materials which are resistant to corrosion as Hastelloy, Inconel, Monel and/or fluoropolymers linings.

Preferably the vessel contains catalyst, for example a fixed or fluid catalyst bed, packed with a suitable fluorination catalyst, with suitable means to ensure that the reaction mixture is maintained with the desired reaction temperature range.

Thus, it is contemplated that the fluorination reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably a chromium-based catalyst (such as Cr₂O₃ catalyst), an iron-based catalyst (such as FeCl₃ on carbon (designated herein as FeCl₃/C for convenience), and combinations of these. In preferred embodiments, the catalyst is a combination of the two aforementioned catalysts, where the reaction vessel contains in a first zone the chromium-based catalyst and in a second zone the iron-based catalyst. The temperature of the reaction in the chromium-based catalyst reaction is preferably kept at a temperature of from 200°C to 600°C and even more preferably from 250°C to 500°C. The temperature of the reaction in the iron-based catalyst reaction zone is preferably kept at a temperature of from 80°C to 300°C and even more preferably from 100°C to 250°C.

In general it is also contemplated that a wide variety of reaction pressures may be used for the fluorination reaction, depending again on relevant factors such as the specific catalyst being used and the most desired reaction product. The reaction pressure can be, for example, superatmospheric, atmospheric or under vacuum, and in certain preferred embodiments is from 7 kPa to 1 MPa absolute (1 to 200 psia), and in certain embodiments from 7 kPa to 0.83 Mpa absolute (1 to 120 psia).

In certain embodiments, an inert diluent gas, such as nitrogen, may be used in combination with the other reactor feed(s).

It is contemplated that the amount of catalyst use will vary depending on the particular parameters present in each embodiment.

### II. FLUORINATION OF THE COMPOUND OF FORMULA (IAA)

The compound of Formula (IAA), preferably produced as described above, can be subjected to further fluorination reaction(s) to produce HCFC-244bb. Preferably this gas phase reaction is at least partially catalyzed.

The fluorination of the compound of Formula (IAA) is preferably carried out under conditions effective to provide a Formula (IAA) conversion of at least about 40%, more preferably at least about 50%, and even more preferably at least about 60%. Further in certain preferred embodiments, the conversion of the compound of Formula (IA) comprises reacting such compound under conditions effective to produce HCFC-244bb at a selectivity of at least about 70%, more preferably at least about 80%, and even more preferably at least about 85%, with selectivities of about 90% or greater being achieved in certain embodiments.

In general, it is possible that this fluorination reaction step can be carried out in the liquid phase or in the gas phase, or in a combination of gas and liquid phases, and it is contemplated that the reaction can be carried out batch wise, continuous, or a combination of these.

For embodiments in which the reaction comprises a liquid phase reaction, the reaction can be catalytic or non-catalytic. Preferably, a catalytic process is used. Lewis acid catalyst, such as metal-halide catalysts, including antimony halides, tin halides, thallium halides, iron halides, and combinations of two or more of these, are preferred in certain embodiments. Metal chlorides and metal fluorides are particularly preferred. Examples of particularly preferred catalysts of this type include SbCl₅, SbCl₃, SbF₅, SnCl₄, TiCl₄, FeCl₃ and combinations of two or more of these.

In preferred gas phase fluorination of Formula (IAA) compounds, the reaction is at least partially a catalyzed reaction, and is preferably carried out on a continuous basis by introducing a stream containing the compound of Formula (IAA) into one or more reaction vessels, such as a tubular reactor. In certain preferred embodiments, the stream containing the compound of Formula (IAA) is preheated to a temperature of from 50°C to 400°C, and in certain embodiments preferably 80°C. In other embodiments, it is preferred that the stream containing the compound of Formula (IAA) is preheated to a temperature of from 150°C to 400°C, preferably 300°C. This steam, preferably after preheating, is then preferably introduced into a reaction vessel (preferably a tube reactor), which is maintained at the desired temperature, preferably from 50°C to 250°C, more preferably from 50°C to 150°C, where it is preferably contacted with catalyst and fluorinating agent, such as HF.

Preferably the vessel is comprised of materials which are resistant to corrosion as Hastelloy, Inconel, Monel and/or fluoropolymers linings.

Preferably the vessel contains catalyst, for example a fixed or fluid catalyst bed, packed with a suitable fluorination catalyst, with suitable means to ensure that the reaction mixture is maintained within about the desired reaction temperature range.

Thus, it is contemplated that the fluorination reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably an Sb-based catalyst, such as catalyst which is about 50wt% SbCl₅/C. Other catalysts which may be used include: from about 3 to about 6 wt% FeCl₃/C; SbF₅/C; about 20 wt% SnCl₄/C; about 23 wt% TiCl₄/C; and activated carbon. Preferably the catalyst comprises Cl₂ and HF pre-treated SbCl₅/C.

In general it is also contemplated that a wide variety of reaction pressures may be used for the fluorination reaction, depending again on relevant factors such as the specific catalyst being used and the most desired reaction product. The reaction pressure can be, for example, superatmospheric, atmospheric or under vacuum and in certain preferred embodiments is from 7 kPa to 1 MPa absolute (1 to 200 psia), more preferably in certain embodiments from 7 kPa to 0.83 MPa absolute (1 to 120 psia).

In certain embodiments, an inert diluent gas, such as nitrogen, may be used in combination with the other reactor feed(s).

It is contemplated that the amount of catalyst use will vary depending on the particular parameters present in each embodiment.

### III. DEHYDROHALOGENATION OF HCFC-244bb

In certain preferred embodiments, the stream containing HCFC-244bb is preheated to a temperature of from 150°C to 400°C, preferably about 350°C, and introduced into a reaction vessel, which is maintained at about the desired temperature, preferably from 200°C to 700°C, more preferably from 300°C to 700°C, more preferably from 300°C to 450°C, and more preferably in certain embodiments from 350°C to 450°C.

Preferably the vessel is comprised of materials which are resistant to corrosion as Hastelloy, Inconel, Monel and/or fluoropolymers linings. The catalyst present in the vessel can for example be a fixed or fluid catalyst bed, packed with a suitable dehydrohalogenation catalyst, with suitable means to heat the reaction mixture to about the desired reaction temperature.

Thus, it is contemplated that the dehydrohalogenation reaction step may be preformed using a wide variety of process parameters and process conditions in view of the overall teachings contained herein.

This reaction step comprises a gas phase reaction in the presence of a carbon- and/or metal-based catalyst (preferably activated carbon), a nickel-based catalyst (such as Ni-mesh), a palladium based catalyst (such as palladium on carbon and palladium on aluminum oxides). Of course, two or more any of these catalysts, or other catalysts not named here, may be used in combination.

The gas phase dehydrohalogenation reaction may be conducted, for example, by introducing a gaseous form of HCFC-244bb into a suitable reaction vessel or reactor. Preferably the vessel is comprised of materials which are resistant to corrosion as Hastelloy, Inconel, Monel and/or fluoropolymers linings. The catalyst can, for example, be a fixed or fluid catalyst bed, packed with a suitable dehydrohalogenation catalyst, with suitable means to heat the reaction mixture to about the desired reaction temperature.

The reaction temperature for the dehydrohalogentation step is from 200°C to 800°C, more preferably from 400°C to 800°C, and even more preferably from 400°C to 500°C, and more preferably in certain embodiments from 300°C to 500°C.

In general it is also contemplated that a wide variety of reaction pressures may be used, depending again on relevant factors such as the specific catalyst being used and the most desired reaction product. The reaction pressure can be, for example, superatmospheric, atmospheric or under vacuum, and in certain preferred embodiments is from 7 kPa to 1 Mpa absolute (1 to 200 psia), and even more preferably in certain embodiments from 7 kPa to 0.83 Mpa absolute (1 to 120 psia).

In certain embodiments, an inert diluent gas, such as nitrogen, may be used in combination with the other reactor feed(s), When such a diluent is used, it is generally preferred that HCFC-244bb comprise from about 50% to greater than 99% by weight based on the combined weight of diluent and HCFC-244bb.

It is contemplated that the amount of catalyst use will vary depending on the particular parameters present in each embodiment.

Preferably in such dehydrofluorination embodiments as described in this section, the conversion of HCFC-244bb is at least about 60%, more preferably at least about 75%, and even more preferably at least about 90%. Preferably in such embodiments, the selectivity to HFO-1234yf is at least about 50%, more preferably at least about 70% and more preferably at least about 80%.

### EXAMPLES

Additional features of the present invention are provided in the following examples, which should not be construed as limiting the claims in any way.

### Example 1 (Reference)

### Preparation of CH₂=CClCH₂Cl (2,3-Dichloro-1-propene) from CH₂ClCHClCH₂Cl

About 8500 grams of 1,2,3-trichloropropane and about 88.0 grams Aliquat 336 were charged into a 30 liter glass vessel, equipped with TEFLON^{®} shaft and stir blades, heated with internal TEFLON^{®} coated copper coils and refrigerant/heating circulation bath and refrigerated condenser. The mixture was then heated to about 73°C with medium speed agitation. At this temperature, about 10,000 grams of 25 wt% NaOH/H₂O solution is added into the reactor from a separate container over a 2 hour period of time. The pH was kept at about 14. After addition, the reaction progress was monitored by GC and GC/MS. The conversion of 1,2,3-trichloropropane was about 97.5% and the selectivity to CH₂=CClCH₂Cl was about 95.4%. After the stipulated reaction time, the mixture was cooled and about 4.0 liters of distilled and ionized water was added into the mixture. The mixture was stirred for about 10 minutes and allowed to separate. The lower layer product (boiling point of about 92.5°C) was drained and distilled to substantially isolate and purity product. The crude yield before distillation was about 6408 grams (GC purity of about 93%).

### Example 2 (Reference)

### Preparation of HCCl₂CCl₂CH₂Cl from CH2=CClCH₂Cl

Chlorine was bubbled into about 82.4 g of 2,3-dichloropxopene at about 10 to about 30 °C with the aid of ice bath cooling until a pale yellow color persisted for about 45 minutes. The crude product in an amount of about 130.4 g, consisted of about 93.6 % CH₂ClCCl₂CH₂Cl and about 2.6 % 2,3-.dichloropropene.

Five hundred grams of CH₂ClCCl₂CH₂Cl was charged into a photoreactor. The jacket for the reactor as well as the jacket for the 450 W UV lamp were cooled to about 15°C using a circulating cooling bath. A total of about 150 g of chlorine was bubbled into the organic liquid over a period of about 2 hours. The crude product weighed about 591 g. GC analysis indicated a conversion of about 54.4 % and selectivity for the desired HCCl₂CCl₂CH₂Cl of about 87 %. Distillation provided HCCl₂CCl₂CH₂Cl in 99 % purity.

### Example 3 (Reference)

### Preparation of CCl₂=CClCH₂Cl from HCCl₂CCl₂CH₂Cl

Aliquat-336^{®} (about 0.26 g) and about 24.8 g of HCCl₂CCl₂CH₂Cl were stirred rapidly at room temperature while adding about 20 g of 25 % aqueous NaOH over 19 minutes. Stirring was continued overnight before adding 30 mL water and allowing the phases to separate. The lower organic phase, in an amount of about 19.8 g, was about 97.5 % pure CCl₂=CC₂CH₂Cl by GC analysis (96 % yield). Prior to fluorination, it was distilled (bp about 69 to about 72 °C at about 4 kPa, 30 mm Hg) to remove any phase transfer catalyst. H NMR: δ 4.41 (s) ppm.

### Example 4 (Reference)

### Selective catalyzed-transformation of CCl₂=CClCH₂Cl to CF₃CCl=CH₂ (HFO-1233xf) in gas-phase

An 56cm (22-inch) long and 1cm (1/2-inch) diameter Monel pipe gas-phase reactor is charged with about 120 cc of a catalyst or a mixture of two catalysts. In case of a mixture, Cr₂O₃ catalyst is kept at the bottom zone of the reactor at a constant temperature of about 270°C-500°C and the other catalyst, such as FeCl₃/C₃ is kept at the middle and the top zone of the reactor at a constant temperature of about 120°C - 220°C. The reactor is mounted inside a heater with three zones (top, middle, and bottom). The reactor temperature is read by custom-made-5-point thermocouples kept inside at the middle of the reactor. The bottom of the reactor is connected to a pre-heater, which is kept at 300°C by electrical heating. The liquid-HF is fed from a cylinder into the pre-heater through a needle valve, liquid mass-flow meter, and a research control valve at a constant flow of about 1 to about 1000 grams pre hour (g/h). The HF cylinder is kept at a constant pressure of 310 kPa gauge (45 psig) by applying anhydrous N₂ gas pressure into the cylinder head space. About 10 to about 1000 g/h of CCl₂=CClCH₂Cl is fed as a liquid through a dip tube from a cylinder under about 310 kPa gauge (45 psig) of N₂ pressure. The organic flows from the dip tube to the preheater (kept at about 250°C) through a needle valve, liquid mass-flow meter, and a research control valve at a constant flow of 1-1000 g/h. The organic is also fed as a gas while heating the cylinder containing organic at about 220°C. The gas coming out of the cylinder is passed through a needle valve and a mass flow controller into the preheater. The organic line from the cylinder to the pre-heater is kept at about 200°C by wrapping with constant temperature heat trace and electrical heating elements. All feed cylinders are mounted on scales to monitor their weight by difference. The catalysts are dried at the reaction temperature over a period of about 8 hours and then pretreated with about 50 g/h of HF under atmospheric pressure over a period of about 6 hours and then under 350 kPa gauge (50 psig) HF pressure over another period of about 6 hours before contacting with organic feed containing CCl₂=CClCH₂Cl. The reactions are run at a constant reactor pressure of about 0 to about 1 MPa gauge (150 psig) by controlling the flow of reactor exit gases by another research control valve. The gases exiting reactor are analyzed by on-line GC and GC/MS connected through a hotbox valve arrangement to prevent condensation. The conversion of CCl₂=CClCH₂Cl is about 70 to about 100% and the selectivity to 1233xf is about 80% to about 95%, respectively. The product is collected by flowing the reactor exit gases through a scrubber solution comprising about 20wt% to about 60 wt% . KOH in water and then trapping the exit gases from the scrubber into a cylinder kept in dry ice or liquid N₂. The product, 1233xf is then substantially isolated by distillation: The results are tabulated in Table 1.

**Table 1: Transformation of CCl₂=CClCH₂Cl to CF₃CCl=CH₂ (CCl₂=CClCH₂Cl + 3HF → CF₃CCl=CH₂ + 3HCl)**

| **#** | **Catalyst** | **T, °C** | **HF. flow, g/h** | **CCl₂=CClCH₂Cl flow, g/h** | **% Conv of CCl₂=CClCH₂Cl** | **% Sel to 1233xf** |
|---|---|---|---|---|---|---|
| **1** | 10% v/v Cr₂O₃-90% v/v FeCl₃/C | 350/150 | 50 | 12 | 79 | 81 |
| **2** | 20% v/v Cr₂O₃-80% v/v FeCl₃/C | 350/150 | 50 | 12 | 83 | 86 |
| **3** | 30% v/v Cr₂O₃-70% v/v FeCl₃/C | 350/150 | 50 | 12 | 89 | 96 |
| **4** | 30% v/v Cr₂O₃-70% v/v FeCl₃/C | 350/150 | 70 | 12 | 79 | 93 |
| **5** | 30% v/v Cr₂O₃-70% v/v FeCl₃/C | 345/170 | 50 | 25 | 85 | 90 |
| **6** | Cr₂O₃ | 350 | 50 | 20 | 90 | 93 |
| **7** | FeCl₃/C | 150 | 50 | 20 | 74 | 39 |

| **#** | **Catalyst** | **T,°C** | **HF flow, g/h** | **CCl₂=CClCH₂Cl flow, g/h** | **% Conv of CCl₂=CClCH₂Cl** | **% Sel to 1233xf** |
|---|---|---|---|---|---|---|
| **8** | SbCl₅/C | 150 | 50 | 20 | 81 | 52 |
| Reaction conditions: Catalyst used (total) 120 cc; pressure, 10 kPa gauge (1.5 psig); | | | | | | |

### Examples 5A and 5B (Reference)

### Liquid-phase catalytic fluorination of CF₃CCl=CH₂ (1233xf) with HF to CF₃CFClCH₃ (244bb)

### Example 5A (Reference)

About 327 grams of HF, about 50 grams 1233xf, and about 75 grams SbCl₅ were charged into a 1-L autoclave. The reaction mixture was stirred at a temperature of about 80°C for about 3 hours under about 4 MPa gauge (about 620 psig) of pressure. After the reaction, the reactor was cooled to about 0°C and about 300 ml water was then added slowly into the autoclave over a period of about 45 min. After complete addition of water under stirring, the reactor was cooled to room temperature and then the overhead gases were transferred to another collecting cylinder. The yield of CF₃CFCFClCH₃ was about 90% at a 1233xf conversion level of about 98%. The other major by-products were CF₃CF₂CH₃ (2%), and an unidentified isomer of a C4 compound of the general formula, C₄H₃Cl₃F₄ (8%).

### Example 5B (Reference)

About 327 grams HF, about 50 grams 1233xf, and about 75 grams SbCl₅ were charged into a 1-L autoclave. The reaction mixture was stirred at 80°C for about 3 hours under about 4 MPa gauge (about 625 psig) of pressure. After the reaction, the reactor was cooled to about 45°C and then the overhead gas mixture was passed through a well dried KF, NaF, or Al₂O₃ (350 g) packed column kept at about 80°C to strip off HF from the gas stream. The gases coming out of the column are collected in a cylinder kept in dry ice (-70°C) bath. The yield of CF₃CFClCH₃ was 87% at a 1233xf conversion level of 93%. The other major by-products were CF₃CF₂CH₃ (1%), and an unidentified isomer of a C4 compound of the general formula, C₄H₃Cl₃F₄ (7%). The product, CF₃CFClCH₃ was isolated by distillation with 98% purity.

### Example 6 (Reference)

### Gas-phase catalytic fluorination of CF₃CCl=CH₂ (1233xf) with HF to CF₃CFClCH₃ (244bb)

A 56 cm (22-inch) (1 cm, 1/2-inch diameter) Monel tube gas phase reactor was charged with about 120 cc of a catalyst. The reactor was mounted inside a heater with three zones (top, middle and bottom). The reactor temperature was read by a custom made 5-point thermocouple kept at the middle inside of the reactor. The inlet of the reactor was connected to a pre-heater, which was kept at about 300°C by electrical heating Organic (1233xf) was fed from a cylinder kept at 70°C through a regulator, needle valve, and a gas mass-flow-meter. The organic line to the pre-heater was heat traced and kept at a constant temperature of about 73°C by electrical heating to avoid condensation. N₂ was used as a diluent in some cases and fed from a cylinder through a regulator and a mass flow controller into the pre-heater. All feed cylinders were mounted on scales to monitor their weight by difference. The reactions were run at a constant reactor pressure of from about 0 to about 700 kPa gauge (about 100 psig) by controlling the flow of reactor exit gases by another research control valve. The gas mixtures exiting reactor was analyzed by on-line GC and GC/MS connected through a hotbox valve arrangements to prevent condensation. The conversion of 1233xf was from about 50% to about 65% and the selectivity to 244 isomer (CF₃CFClCH₃) was from about 90% to about 93% depending on the reaction conditions using 120 cc of 50 wt% SbCl₅/C as the catalyst at about 65°C to about -85°C with a HF flow of about 50 g/h and organic flow of about 15 g/h. No CF₃CF₂CH₃ was observed under the reaction conditions. The catalyst is pretreated at first with 50 g/h HF at about 65°C for about 2 hours and then with about 50 g/h HF and about 200 sccm of Cl₂ at about 65°C for about 4 hours. After pretreatment, about 50 sccm of N₂ is flows over a period of about 40 minutes through the catalyst bed to sweep free chlorine from the catalyst surface prior to interacting with the organic feed (1233xf). Pretreatment is considered important to many embodiments of the invention. The products were collected by flowing the reactor exit gases through a 20-60 wt% aqueous KOH scrubber solution and then trapping the exit gases from the scrubber into a cylinder kept in dry ice or liquid N₂. The products were then isolated by distillation. About 50 wt% SbCl₅/C, about 3 to about 6 wt% FeCl₃/C, , 20 wt% SnCl₄/C, and about 23 wt% TiCl₄/C, using 4 different kind of activated carbon such as Shiro saga, Calgon, Norit, and Aldrich were used as the catalyst at from about 60 to about 150°C. Among all the catalysts used for this reaction, Cl₂ and HF pre-treated SbCl₅/C was found to be generally preferred in terms of activity. The results using SbCl₅ as the catalyst are shown in Table 2.

**Table 2: Catalyzed-gas-phase transformation of CF₃CCl=CH₂ to CF₃CFClCH₃**

| # | Cat | T, °C | Conv. of CF₃CCl=CH₂ (1233xf) | Sel. to CF₃CFClCH₃ |
|---|---|---|---|---|
| 1 | 10 wt% SbCl₅/C | 60 | 15 | 100 |

| # | Cat | T, °C | Conv. of CF₃CCl=CH₂ (1233xf) | Sel. to CF₃CFClCH₃ |
|---|---|---|---|---|
| 2 | 20 wt% SbCl₅/C | 60 | 21 | 98 |
| 3 | 30 wt% SbCl₅/C | 60 | 32 | 98 |
| 4 | 50 wt% SbCl₅/C | 60 | 55 | 97 |
| 5 | 50 wt% SbCl₅/C | 80 | 62 | 93 |
| 6 | 50 wt% SbCl₅/C | 100 | 56 | 87 |
| 7 | 60 wt% SbCl₅/C | 60 | 59 | 91 |
| 8 | 50 wt% SbCl₅/NORIT RFC 3 Activated Carbon | 60 | 34 | 92 |
| 9 | 50 wt% SbCl₅/Shiro Saga Activated Carbon | 60 | 56 | 96 |
| 10 | 50 wt% SbCl₅/Aldrich Activated Carbon | 60 | 57 | 94 |

Reaction conditions: 1233xf flow, 150 sccm; HF flow 50 g/h; pressure, 17-37 kPa gauge (2.5-5.3 psig); in 1-5 reactions Calgon activated carbon is used as the catalyst support; catalyst, 120 cc. All catalysts are pre-treated with Cl₂ and HF prior to contacting with 1233xf.

### Example 7

### Conversion of CF₃CFClCH₃ to CF₃CF=CH₂ in gas-phase

A 56cm (22-inch) (1 cm, 1/2-inch diameter) Monel tube gas phase reactor was charged with 120 cc of catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic (CF₃CFClCH₃) was fed from a cylinder kept at about 65°C through a regulator, needle valve, and a gas mass-flow-meter. The organic line to the pre-heater was heat traced and kept at a constant temperature of from about 65°C to about 70°C by electrical heating to avoid condensation. The feed cylinder was mounted on scales to monitor their weight by difference. The reactions were run at a constant reactor pressure of from about 0 to about 700 kPa gauge (about 100 psig) by controlling the flow of reactor exit gases by another research control valve. The gas mixture exiting reactor was analyzed by on-line GC and GC/MS connected through a hotbox valve arrangement to prevent condensation. The conversion of CF₃CFClCH₃ was almost 98% and the selectivity to HFO-1234yf was from about 69% to about 86% depending on the reaction conditions. The products were collected by flowing the reactor exit gases through a about 20wt% to about 60 wt% of aquesous KOH scrubber solution and then trapping the exit gases from the scrubber into a cylinder kept in dry ice or liquid N₂. The products were then isolated by distillation. Results are tabulated in Table 3.

**Table 3: Catalyzed-transformation of CF₃CFClCH₃ to HFO-1234yf**

| # | Cat | T, °C | | Flow rate, CF₃CFClCH₃ (244 isomer) sccm | Conversion of 244 | 1234yf (Sel. %) |
|---|---|---|---|---|---|---|
| 1 | A | 400 | | 150 | 100 | 46 |
| 2 | B | 400 | | 150 | 96 | 63 |
| 3 | C | 400 | | 100 | 100 | 64 |
| 4 | D | 400 | | 100 | 99 | 93 |
| 5 | D | 400 | | 150 | 92 | 89 |
| 6 | E | 400 | | 100 | 96 | 56 |
| 7 | F | 400 | | 100 | 87 | 51 |
| 8 | G | 400 | | 100 | 100 | 37 |

**Reaction conditions:** pressure, 17-37 kPa gauge (2.5-5.3 psig); catalyst, 100 cc, A is NORIT RFC 3; B is Shiro-Saga activated carbon; C is Aldrich activated carbon; D is Calgon activated carbon; activated carbon; E is 0.5 wt% Pd/C; F is 0.5 wt% Pt/C; G is Ni-mesh; Organic cylinder temperature-65°C; CF₃CFClCH₃ (244) line to the preheater-60°C; Preheater, 350°C; P-5 psig.

### Example 8 (Reference)

### Selective catalyzed-transformation of CCl₃CCl=CH₂ to CF₃CCl=CH₂ (HFO-1233xf) in gas-phase

A 56cm (22-inch) long and 1cm (1/2-inch) diameter Monel pipe gas phase reactor was charged with 120 cc of a catalyst or a mixture of two catalysts. In case of a mixture, Cr₂O₃ catalyst is kept at the bottom zone of the reactor at a substantially constant temperature of from about 270°C to about 500°C and the other catalyst, such as FeCl₃/C is kept at the middle and the top zone of the reactor at a substantially constant temperature of from about 120°C to about 220°C. The reactor was mounted inside a heater with three zones (top, middle, and bottom). The reactor temperature was read by custom-made-5-point thermocouples kept inside at the middle of the reactor. The bottom of the reactor was connected to a pre-heater, which was kept at about 300°C by electrical heating. The liquid-HF was fed from a cylinder into the pre-heater through a needle valve, liquid mass-flow meter, and a research control valve at a substantially constant flow of from about 1 to about 1000 g/h. The HF cylinder was kept at a substantially constant pressure of about 310 kPa (about 45 psig) by applying anhydrous N₂ gas pressure into the cylinder head space. A feed rate of from about 10 g/h to about 1000 g/h of CCl₃CCl=CH₂ was fed as a liquid through a dip tube from a cylinder under about 310 kPa gauge (about 45 psig) of N₂ pressure. The organic was flown from the dip tube to the pre-heater (kept at about 250°C) through needle valve, liquid mass-flow meter, and a research control valve at a substantially constant flow of from about 1 to about 1000 g/h. The organic is also fed as a gas while heating the cylinder containing organic at about 220°C. The gas effluent from the cylinder is passed through a needle valve and a mass flow controller into the pre-heater. The organic line from the cylinder to the pre-heater was kept at about 200°C by wrapping with constant temperature heat trace and electrical heating elements. All feed cylinders were mounted on scales to monitor their weight by difference. The catalysts were dried at the reaction temperature over a period of about 8 hours and then pretreated with about 50 g/h of HF under atmospheric pressure over a 6 hour period and then under about 350 kPa gauge (about 50 psig) HF pressure over a 6 hour period before contacting with organic feed, CCl₃CCl=CH₂. The reactions were run at a substantially constant reactor pressure ranging from about 0 to about 1 MPa gauge (about 150 psig) by controlling the flow of reactor exit gases by another research control valve. Those gases exiting reactor were analyzed by on-line GC and GC/MS connected through a hotbox valve arrangements to prevent condensation. The conversion of CCl₃CCl=CH₂ was in a range of from about 90% to about 100% and the selectivity to CF₃CCl=CH₂ (1233xf) was about 79%.. The effluent contained in addition HFO-1243zf in an amount of about 7.7%, 1232-isomer in an amount of about 1.3%, and 1223 in an amount of about 0.8%, and an unidentified byproduct. The product was collected by flowing the reactor exit gases through a 20-60 wt% aq. KOH scrubber solution and then trapping the exit gases from the scrubber into a cylinder kept in dry ice or liquid N₂. The product, 1233xf was then substantially isolated by distillation. Using only Cr₂O₃ catalyst, a selectivity of about 68% to 1233xf at a conversion level of about 79% was achieved.

### Examples 9A - 9D (Reference)

### Direct Liquid-phase catalytic fluorination of CCl₃CCl=CH₂ with HF to CF₃CFClCH₃ (244-isomer)

### Example 9A

About 327 grams HF, about 50 grams CCl₃CCl=CH₂, and about 75 grams SbCl₅ were charged into a 1-L autoclave. The reaction mixture was stirred at about 80°C for about 3 hours under about 4.2 MPa (about 610 psig) of pressure. After the reaction, the reactor was cooled to about 40°C and about 300 ml water was then added slowly into the autoclave over a period of about 45 min. After complete addition of water under stirring, the reactor was cooled to about room temperature and then the overhead gases were transferred to another collecting cylinder. The yield of CF₃CFClCH₃ was about 89% at a CCl₃CCl=CH₂ conversion level of about 88%. The other major by-products were CF₃CF₂CH₃ (2%), and an unidentified isomer of a C4 compound of the general formula, C₄H₃Cl₃F₄ (8%).

### Example 9B

About 327 grams HF, about 50 grams CCl₃CCl=CH₂, and about 75 grams SbCl₅ were charged into a 1-L autoclave. The reaction mixture was stirred at about 100°C for about 3 hours under about 4.72 MPa gauge (about 685 psig) of pressure. After the reaction, the reactor was cooled to about 40°C and about 300 ml water was then added slowly into the autoclave over a period of about 45 minutes. After complete addition of water, under stirring, the reactor was cooled to room temperature and then the overhead gases were transferred to another collecting cylinder. The yield of CF₃CFClCH₃ was about 78% at a CCl₃CCl=CH₂ conversion level of about 100%. The other major by-products were CF₃CF₂CH₃ (about 4%), and an unidentified isomer of a C4 compound of the general formula, C₄H₃Cl₃F₄ (about 13%).

### Example 9C

About 327 grams HF, about 50 grams CCl₃CCl=CH₂, and about 75 grams SbCl5 were charged into a 1-L autoclave. The reaction mixture was stirred at about 125°C for about 6 hours under about 825 psig of pressure. After the reaction, the reactor was cooled to about 40°C and about 300 ml water was then added slowly into the autoclave over a period of about 45 min. After complete addition of water under stirring, the reactor was cooled to about room temperature and then the overhead gases were transferred to another collecting cylinder. The major products were CF₃CF₂CH₃ (about 53%) and CF₃CFClCH₃ (about 25%) at a CCl₃CCl=CH₂ conversion level of about 100%. The other major by-products were and unidentified isomer of a C4 compound of the general formula, C₄H₃Cl₃F₄ (8%) and tar.

### Example 9D

About 327 grams HF, about 50 grams CCl₃CCl=CH₂, and about 75 g SbCl₅ were charged into a 1-L autoclave. The reaction mixture was stirred at about 150°C for about 6 hours under about 825 psig of pressure. After the reaction, the reactor was cooled to about 40°C and about 300 ml water was then added slowly into the autoclave over a period of about 45 minutes. After complete addition of water under stirring, the reactor was cooled to about room temperature and then the overhead gases were transferred to another collecting cylinder. The major products were CF₃CF₂CH₃ (about 57%) and CF₃CFClCH₃ (about 15%) at a CCl₃CCl=CH₂ conversion level of about 100%. The other major by-products were and unidentified isomer of a C4 compound of the general formula, C₄H₃Cl₃F₄ (about 11%) and tar.

### Example 10 (Reference)

### Catalytic conversion of CF₃CF₂CH₃ to CF₃CF=CH₂

A 56cm (22-inch) (1 cm, 1/2-inch diameter) Monel tube gas phase reactor was charged with 120 cc of a catalyst. The reactor was mounted inside a heater with three zones (top, middle and bottom). The reactor temperature was read by custom made 5-point thermocouples kept at the middle inside of the reactor. The inlet of the reactor was connected to a pre-heater, which was kept at about 300°C by electrical heating. Organic material (245cb) was fed from a cylinder kept at about 65°C through a regulator, needle valve, and a gas mass-flow-meter. The organic line to the pre-heater was heat traced and kept at a substantially constant temperature in a range of from about 65°C to about 70°C by electrical heating to avoid condensation. The feed cylinder was mounted on a scale to monitor its weight by difference. The reactions were run at a substantially constant reactor pressure of from about 0 to about 0.7 MPa gauge (100 psig) by controlling the flow of reactor exit gases by another research control valve. The gas mixtures exiting reactor was analyzed by on-line GC and GC/MS connected through a hotbox valve arrangements to prevent condensation. The conversion of 245cb was in the range of from about 30% to about 70% and the selectivity to 1234yf was in the range of from about 90% 5o about 100% depending on the reaction conditions. The products were collected by flowing the reactor exit gases through a 20-60-wt% of aq. KOH scrubber solution and then trapping the exit gases from the scrubber into a cylinder kept in dry ice or liquid N₂. The products were then substantially isolated by distillation. Results are tabulated in Table 4.

**Table 4: Transformation of CF₃CF₂CH₃ to 1234yf**

| # | Cat | T, °C | H₂, sccm | CF₃CF₂CH₃ (245cb) sccm | Conversion of 24Scb, % | 1234yf (Sel. %) |
|---|---|---|---|---|---|---|
| 1 | A | 575 | 0 | 65 | 79 | 63 |
| 2 | B | 575 | 0 | 68 | 82 | 57 |
| 3 | C | 575 | 0 | 73 | 73 | 61 |
| 4 | D | 575 | 0 | 68 | 84 | 59 |
| 5 | D | 575 | 20 | 68 | 89 | 73 |
| 6 | E | 550 | 0 | 69 | 92 | 53 |
| 7 | F | 550 | 0 | 67 | 93 | 33 |
| 8 | G | 550 | 0 | 69 | 73 | 46 |
| **Reaction conditions:** pressure, 17-37 kPa gauge (2.5-5.3 psig); catalyst, 100 cc, A is NORIT RFC 3; B is Shiro-Saga activated carbon; C is Aldrich activated carbon; D is Calgon activated carbon; E is 0.5 wt% Pd/C; F is 0.5 wt% Pt/C; G is Ni-mesh; Organic cylinder temperature is about 65°C; CF₃CF₂CH₃ (245cb) line to the preheater is maintained at about 50°C; preheater temperature is maintained at about 350°C; N₂ flow is not used; pressure is maintained at about 20 kPa gauge (3 psig). | | | | | | |

## Claims

1. A method for producing fluorinated organic compounds comprising converting 1,1,1,2-tetrafluoro-2-chloropropane (HCFC-244bb) into 2,3,3,3-tetrafluoro-1-propene (HFO-1234yf);
wherein the reaction is carried out in a gas phase in the presence of a catalyst selected from the group consisting of carbon and/or metal based catalyst, nickel based catalyst, and palladium based catalyst, and wherein the reaction temperature is from 200°C to 800°C.

2. The method of claim 1, wherein the reaction temperature is from 400°C to 800°C.

3. The method of claim 1, wherein the reaction temperature is from 400°C to 500°C.

4. The method of claim 1, wherein the reaction temperature is from 300°C to 500°C.

## Patentansprüche

1. Verfahren zur Herstellung von fluorierten organischen Verbindungen, bei dem man 1,1,1,2-Tetrafluor-2-chlorpropan (H-FCKW 244bb) in 2,3,3,3-Tetrafluor-1-propen (HFO-1234yf) umwandelt;
wobei die Umsetzung in einer Gasphase in Gegenwart eines Katalysators aus der Gruppe bestehend aus Katalysatoren auf Kohlenstoff- und/oder Metallbasis, Katalysatoren auf Nickelbasis und Katalysatoren auf Palladiumbasis durchgeführt wird und wobei die Reaktionstemperatur 200 °C bis 800 °C beträgt.

2. Verfahren nach Anspruch 1, bei dem die Reaktionstemperatur 400 °C bis 800 °C beträgt.

3. Verfahren nach Anspruch 1, bei dem die Reaktionstemperatur 400 °C bis 500 °C beträgt.

4. Verfahren nach Anspruch 1, bei dem die Reaktionstemperatur 300 °C bis 500 °C beträgt.

## Revendications

1. Procédé pour la production de composés organiques fluorés comprenant la conversion de 1,1,1,2-tétrafluoro-2-chloropropane (HCFC-244bb) en 2,3,3,3-tétrafluoroprop-1-ène(HFO-1234yf) ;
dans lequel la réaction est effectuée en phase gazeuse en présence d'un catalyseur choisi dans le groupe constitué par un catalyseur à base de carbone et/ou de métal, un catalyseur à base de nickel et un catalyseur à base de palladium, et dans lequel la température de la réaction est de 200 °C à 800 °C.

2. Procédé selon la revendication 1, dans lequel la température de la réaction est de 400 °C à 800 °C.

3. Procédé selon la revendication 1, dans lequel la température de la réaction est de 400 °C à 500 °C.

4. Procédé selon la revendication 1, dans lequel la température de la réaction est de 300 °C à 500 °C.
